Europäisches Patentamt

European Patent Office

Office européen des brevets

(11) Publication number: **0 282 832**
**A1**

## EUROPEAN PATENT APPLICATION

(21) Application number: **88103314.6**

(51) Int. Cl.⁴: **A61B 1/24**

(22) Date of filing: **03.03.88**

(30) Priority: **05.03.87 US 22170**
**23.07.87 US 77367**

(43) Date of publication of application:
**21.09.88 Bulletin 88/38**

(34) Designated Contracting States:
**DE ES FR GB IT SE**

(71) Applicant: **HIGH-TECH MEDICAL**
**INSTRUMENTATION INC.**
**P.O. Box 2984**
**Dublin California 94568(US)**

(72) Inventor: **Milbank, Miles Christian**
**9540 Sand Point Drive**
**San Ramon California 94583(US)**
Inventor: **Williams, Perry Michael**
**917 Walnut Street**
**San Carlos California 94070(US)**

(74) Representative: **Dipl.-Phys.Dr. Manitz**
**Dipl.-Ing., Dipl.-Wirtsch. Finsterwald Dipl.-Ing.**
**Grämkow Dipl.-Chem.Dr. Heyn Dipl.-Phys.**
**Rotermund**
**Morgan, B.Sc.(Phys.) Robert-Koch-Strasse 1**
**D-8000 München 22(DE)**

(54) **Dental device for intraoral video imaging.**

(57) A digitally manipulable tool for creating video images of the inside of a patient's mouth is disclosed. The tool comprises a small video head (14) carried on the distal end of a lightweight, digitally manipulable support structure (10). In addition to enabling the nonrelative, intraoral positioning of the head, the support structure also provides a conduit (18) for carrying electrical and optical signals to and from the head. The tool may be used with conventional endoscopic processing and monitoring equipment for visual display and recording. In a modification, a cover (27) for the head is semi-reflective to permit simultaneous imaging and viewing by the user. In another modification, a protective sheath covers (28) the distal end of the tool to prevent contamination of the head.

FIG._1.

## DENTAL DEVICE FOR INTRAORAL VIDEO IMAGING

### BACKGROUND OF THE INVENTION

#### Field of the Invention

This invention relates generally to dental apparatus, and more particularly to a hand-held, easily manipulated tool with a signal-producing imaging head on one end and a cord on the other. While, as will be expressed somewhat more fully below, we recognize that various spectrum imaging devices, such as those involving the radio spectrum, the infrared spectrum, the area of fluorescence, and the area of sound, may be used, a preferred embodiment of the invention herein is described in conjunction with a video imaging device which has been found to offer particularly broad utility in the field of dentistry.

#### Description of the Prior Art

Endoscopy is a well known technique for viewing internal body organs that are not otherwise viewable. Such viewing is accomplished by introducing into a body cavity a flexible fiberscope capable of optically communicating picture element (pixel) information to external video imaging equipment. The video imaging equipment typically includes a lens and a camera, and is connected to a video processing system having recording and monitoring capabilities. Thus, a video image of a relatively inaccessible region of the body can be viewed live or from a taped record. Video image resolution and the endoscopy apparatus of the prior art is limited by the number of fiber optic elements that can be bundled into a flexible fiberscope.

Advances in the microminiaturization of charge coupled devices (CCDs) have made possible the replacement of the fiberscope, and external lens and camera with a video imaging circuit device at the tip end of a smaller and more flexible electro-optical handpiece. This endoscopic probe has enabled deeper penetration and viewing of internal regions of the body previously unexplored. The advent of monolithic CCD arrays has also improved the image resolution attainable by endoscopic imaging techniques.

Endoscopic techniques are traditionally employed in medical practice. Some of the difficulties encountered in using endoscopic equipment include: inadequate maneuverability of the operative, distal end of a flexible probe from its relatively controllable proximal end; inadequate positionability of an imaging field that is not directly viewable by the user; and inadequate illumination of the video imaging target area. Despite these drawbacks, endoscopy has extended visual access to the inner reaches of the body, and is widely used in medical research and practice.

In the practice of dentistry, the X-ray machine has been the predominant imaging tool used to record and view dental and periodontal structure. Hand-held tools having mirrored surfaces provide visual access by the dentist to many, but not all areas of the mouth not directly viewable. Such tools, of course, have the further limitation that they provide only a small image, and, were such desired, they offer no capability to provide a permanent record of what the dentist sees. Video imaging and monitoring of these regions of the mouth neither directly viewable, nor indirectly viewable by use of mirrored tools, would greatly expand the dentist's visual field. The ability to monitor what the dentist sees and does while working on a person's teeth would also provide unprecedented educational and training opportunities to students of dentistry and others, e.g., dental hygienists and patients. Soft tissues or surface features that are imageable would be recordable as well, thus augmenting the patient's dental history and assisting in dental diagnosis and treatment.

### SUMMARY OF THE INVENTION

The present invention discloses a hand-held tool that enables the dentist to produce a video image of the desired region of a patient's mouth. When used in conjunction with the video processing equipment mentioned above, the video image produced by this dental tool may be large-scale monitored and/or recorded.

A principal object of the invention is to provide a dental tool that may easily be manipulated by a dentist to produce a video image of a desired region of a patient's mouth.

Another important object of the invention is to provide such a tool in a form that is comfortable and familiar to the dentist.

A further object is to provide such a tool that allows concurrent viewing and video imaging.

Still another object of the invention is to provide a tool of the type outlined that is compatible with conventional endoscopic processing equipment.

According to a preferred embodiment of the invention, a video head containing illuminating and imaging means is carried at the distal end of a hollow, rigid, cylindrical handle. Opto-electronic wiring is sealingly enclosed in the hollow of the handle, and provides power to the video head circuitry, illumination of the subject region, and transmission of the video signal from the head to external endoscopic processing equipment. The gripping surface of the handle is textured to facilitate manipulation of the tool. An obtuse angle formed by the video head and the handle is within the range of angles provided by a variety of conventional, mirrored dental tools. The video head takes the form of a conventional CCD imaging device and a conventional illumination source, e.g., fiber optic bundles transmitting light sourced by the endoscopic processing equipment. The imaging and illuminating circuitry is sealingly overlaid by a polished, transparent, planar cover of e.g., optical-quality glass or plastic.

In a modification to the preferred embodiment of the invention, the cover of the video head is treated in such a manner that it exhibits semi-reflective mirror characteristics. By this modification, while operable for imaging, the tool may also be used by the dentist as a mirror.

In another modification to the preferred embodiment of the invention, a protective, optical-quality sheath is provided for the distal end of the tool to protect the head from cross-patient contamination. The sheath, which may be made of transparent, conformable material such as polyethylene, may be strain-relieved as necessary to secure it to the tool's handle and to conform it to the tool's contour.

In a further modification to the preferred embodiment of the invention, a malleable or deformable member connects the tool's rigid handle and video head to allow variation of the position of the video head relative to the handle. Alternatively, pivots or positionable joints may be provided between the tool's rigid handle and video head to achieve a similar result.

By the unique structure of the dental tool disclosed herein, visual access to the inside of a person's mouth is greatly enhanced by the fact, inter alia, that a dentist can gain a large-scale view on a video screen. The dentist is also given the opportunity to record what is seen. By the first proposed modification to the preferred embodiment of this invention, a targeted region of the mouth may both be imaged on a screen, and viewed directly (by mirror reflection) by the dentist.

These and other advantages and features of the invention will become more fully apparent when the detailed description below is read with reference to the accompanying drawings.

## DESCRIPTION OF THE DRAWINGS

Fig. 1 is a side elevation of a preferred embodiment of the dental tool disclosed herein.

Fig. 2 is a top view of the tool.

Fig. 3 is an end view in the plane of the video head of the tool.

Fig. 4 is a front elevation of the video head corresponding to Fig. 3.

Fig. 5 is similar to Fig. 3, except that it shows a modification to the preferred embodiment of the invention, wherein the video head's cover is specially treated.

Fig. 6 is similar to Fig. 1, except that it shows a further modification of the invention, wherein a protective sheath covers the distal end of the tool.

## DETAILED DESCRIPTION OF THE INVENTION

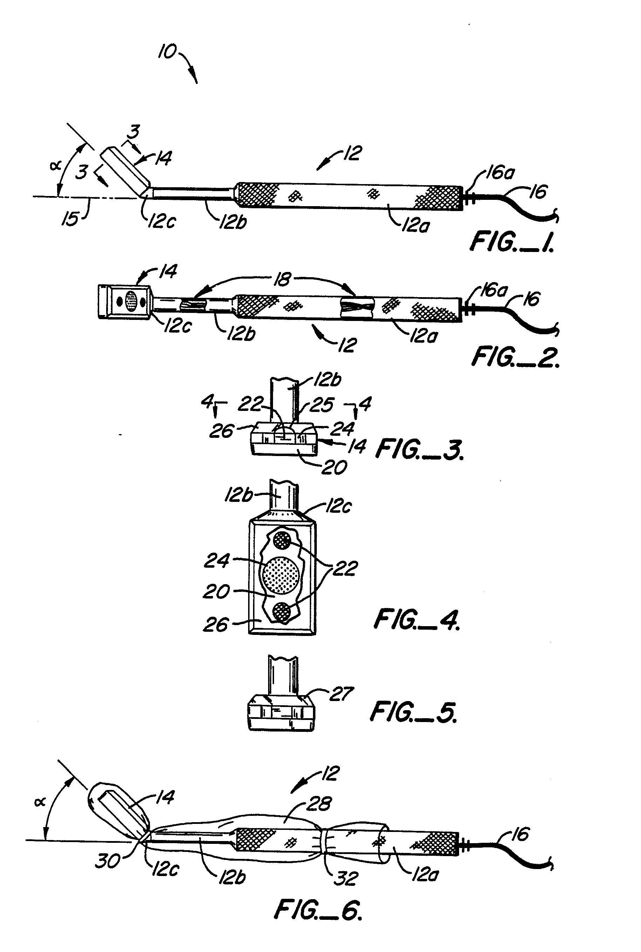

Referring first to Fig. 1, a video imaging tool constructed in accordance with the present invention is indicated at 10. It includes a handle, or support structure, 12 and a video head 14. Head 14, whose structure will be elaborated below, includes a microelectronics-based video imaging device, and a fiber-optics-based source of illumination. Handle 12 has a cross-hatch textured section 12a, a smooth section 12b, and a neck 12c. Head 14 is carried by neck 12c at an angle $\alpha$ to the long axis 15 of handle 12. Angle $\alpha$ herein is about 45°, which typifies the corresponding angle found in conventional dental mirrors.

A cord 16 carries opto-electrical wiring to and from conventional endoscopic processing equipment (not shown). A strain relief 16a is provided to facilitate manipulation of the tool while relieving strain on the wiring.

An important feature of the invention is that handle 12 is firmly joined to head 14 so that manipulation of the handle end of the tool results in one-to-one, nonrelative movement of the video head as a unit with the handle. Tool 10 is light in weight so that a dentist may maneuver it as easily as if it were a mirror or any other familiar, intraoral dental tool.

Referring next to Fig. 2, shown at 18 in cutaways from handle 12 is the "wiring" necessary to allow the video head to communicate, electrically and optically, with the endoscopic processing equipment through cable 16. This "wiring" includes electrical conductors and fiber optic bundles, and provides thereby (1) illumination of the target by ends of the fiber optic bundles on the video head, (2) power to operate the imaging device's microelectronics and (3) an opto-electrical signal conductor for transmitting video imaging information to the endoscopic processing equipment. It will be appreciated that a greater or lesser number of "wires" may be used to perform the necessary functions within the scope of the invention. For example, if the video head were equipped with an integral, self-contained light source (e.g., bulb), the fiber optic bundles would not be needed. Additionally the need for cable 16 would be obviated where wireless remote transmission of video signals (i.e., infra-red remote) is used.

Turning next to Fig. 3, which should be viewed now along with Fig. 2, head 14, generally speaking, includes a mounting substrate 20, the distal end 22 of two fiber optic bundles, which are the bundles mentioned just above, a commercially available, miniature, microelectronics-based charge-coupled video imaging device 24, an objective lens, 25, of one or more elements and a cover 26. As can be seen particularly in Figs. 2 and 4, the ends of bundles 22 are spaced substantially symmetrically on opposite sides of device 24. These bundles, which extend as has been mentioned previously through handle 12 and cable 16, are furnished conventionally with light from a remote light source (not shown). Power for device 24 is supplied remotely, and the video signal produced by the device is transmitted remotely, as mentioned earlier, through handle 12 and cable 16.

Imaging device 24, which actually takes the form of a hybridized high-resolution, monolithic array of light-sensitive CCDs is available, for example, from Promex, Inc., of Mountain View, California. Cover 26, in the embodiment presently being described, takes the form of a bidirectionally transmissive, optical quality plastic which both protects device 24 and the ends of bundles 26, while at the same time allowing these structures to communicate optically with the field of view of the head.

Thus, a novel dental tool is described by which intraoral video imaging may be accomplished. The handle and video head arrangement provide an easily manipulable dental tool that extends and greatly expands visual access to the mouth. The tool is lightweight, and is easily positioned for dental and periodontal inspection by the dentist or oral hygienist who is familiar with the use of similarly shaped and sized tools. Significantly, the invention enables large-screen monitoring of that which was heretofore, if viewable at all, then viewable only in the face of a very small mirror. The tool thus greatly enhances a user's opportunity to view in detail a small work region within a patient's mouth. Further, it readily enables other parties, for example, students in a teaching situation, simultaneously to view a given procedure.

Certainly, an important additional advantage offered by the tool described herein is that it produces an easily recorded signal so that any feature or procedure which a user may wish to capture for later review or for some other use can be recorded conveniently on video tape, for example.

With the video head described herein firmly anchored to the handle which is the structure per se manipulated directly by a user's hand, the one-to-one, move-as-a-unit relationship between these components facilitates maneuvering and placement of the video heads so that a user can repeatedly and reliably and with little effort "home in" on the particular area which he or she wishes to view. It should be apparent, however, that a deformable member may be utilized at 12b of Fig. 1 to enhance the versatility of the tool without jeopardizing the manipulability of the head and handle. Alternatively, neck 12c may be of such pivotable or positionable construction to allow positioning of head 14 relative to handle 12 while maintaining their move-as-a-unit relationship.

As was mentioned earlier herein, other kinds of signal-producing spectrum imaging devices, that is devices other than a video device, may be incorporated in the same kind of structure which we have just above described. Such other kinds of devices, which are useful in specific applications and procedures, when incorporated in the device of the invention, offer the same generic types of advantages which the device just described above offers. As an illustration, if a fluoroscopic procedure is involved, a dental tool of the type set forth above would include in its head, a suitable sensor which is sensitive to the selected fluoroscopic wave length, along with an appropriate source of illumination, which could well be fiber optic bundles, coupled to an appropriate external source. An infrared imaging device would be similarly constructed with suitable illumination and reception devices in a tool head.

Turning now to Fig. 5, a modification to the preferred embodiment of the invention is illustrated. Fig. 5 is identical to Fig. 3, except that here there is a cover 27 which is specially treated, as by coating, to function as a partially reflective mirror, except over the zones directly faced by the distal ends of bundles 22. With this structure, light reflected from the illuminated region of a patient's mouth is both transmitted by the semi-reflective portion of cover 27 onto device 24, and reflected into the eye of the tool's user.

Fig. 6 is identical to Fig. 1, except that it shows another modification to the preferred embodiment of the invention. This modification consists of a protective, transparent, optical-quality, conformable sheath 28 covering the distal end of the tool and secured by a plurality of ties 30, 32. The material of this sheath may be polyethylene or any other, preferably impermeable, optically clear plastic. The purpose of the sheath is to prevent cross-patient contamination as a consequence of contamination of the video head by, e.g., intraoral fluid. It will be appreciated that the protective sheath may take many forms to accomplish its purpose. It will be further appreciated that the ties may be unnecessary or of a different number or arrangement to accomplish their intended purpose.

The advantages offered by the invention should be apparent to those skilled in the art. A lightweight, digitally manipulable dental tool provides the capability of intraoral imaging. In one modification to the preferred embodiment of the invention, the imaging head's cover is treated to transform it into a semi-reflective mirror, enabling both reflective viewing and video imaging of a region in a patient's mouth. Thus, a device capable of signal-producing imaging is uniquely and firmly coupled to a maneuvering handle to enhance a user's ability to view a patient's mouth.

Accordingly, while a preferred embodiment of the invention and modifications thereof have been described herein, it is appreciated that further modifications are possible that come within the scope of the invention.

**Claims**

1. A dental imaging tool comprising:

signal-producing, spectrum imaging means capable of operating within a patient's mouth; and

a rigid, hand-manipulable support structure carrying said imaging means at an obtuse angle to the long axis of said structure, and in a manner enabling movement of the latter as a unit with the former.

2. A dental video imaging tool comprising:

video imaging means capable of operating within a patient's mouth; and

a rigid, hand-manipulable support structure carrying said imaging means at an obtuse angle to the long axis of said structure, and in a manner enabling movement of the latter as a unit with the former.

3. A dental video imaging tool usable with endoscopic processing equipment comprising:

a video head fittable and maneuverable within a patient's mouth; and

a manipulable, rigid support structure carrying said head at an obtuse angle to the long axis of said structure and enabling the intraoral positioning of said head nonrelative to said support structure for video imaging of such patient's mouth.

4. The tool of claim 3, further being fittable with light-reflecting means enabling simultaneous imaging and viewing of a region of the mouth.

5. The tool of claim 3, further comprising means for sealingly protecting said head in such a manner that it is maintained free of contaminants.

6. The tool of claim 3, wherein said support structure is elongate and includes a proximal end, and a distal end carrying said head.

7. The tool of claim 6, wherein said support structure includes a through-bore from said proximal end to said distal end, and wherein said video head sealingly engages said distal end with said through-bore providing a conduit from said head to said proximal end.

8. A dental video imaging tool comprising:

a video head fittable and maneuverable within a patient's mouth; and

a digitally manipulable, elongate support structure having a cylindrical body with a generally concentric cylindrical bore therethrough, from a proximal end to a distal end, said body carrying said head at an obtuse angle to the long axis of said structure, said structure enabling the one-to-one movement of said head nonrelative to said structure, whereby digital manipulation of the structure effects the intraoral positioning of said head for the video imaging of such patient's mouth.

9. The tool of claim 8, further comprising means for sealingly protecting said head in such a manner that it is maintained free of contaminants.

10. The tool of claim 8 that is usable with endoscopic processing equipment, further comprising first means for conducting power from such equipment to said head, and second means for conducting video signals from said head to such equipment.

11. The tool of claim 10, further comprising means for transmitting light from such equipment to said head, and means for directing such light from said head to a region of such patient's mouth.

12. The tool of claim 10, wherein said bore is dimensioned for receiving said first and said second conducting means.

13. The tool of claim 12, further comprising means for illuminating a region of such patient's mouth.

FIG.__1.

FIG.__2.

FIG.__3.

FIG.__4.

FIG.__5.

FIG.__6.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 122 537 (SUMITOMO ELECTRIC INDUSTRIES) <br> * page 2, line 2 - page 7, line 18; page 7, line 19 - page 11, line 8; figures 2, 3, 4 * | 1-3 | A 61 B 1/24 |
| A | | 4,12,13 | |
| A | DE-A-3 045 162 (HEITLINGER) <br> * page 7, lines 1-25; figures 1, 2 * | 1-3,11, 12 | |
| Y | US-A-4 601 284 (ARAKUWA et al.) <br> * abstract; column 3, line 42 - column 5, line 43; figures 1-6 * | 1-3 | |
| A | | 7,10,11 | |

TECHNICAL FIELDS SEARCHED (Int. Cl.4)

A 61 B 1/00

The present search report has been drawn up for all claims

| Place of search | Date of completion of the search | Examiner |
|---|---|---|
| BERLIN | 19-05-1988 | WEIHS J.A. |